Europäisches Patentamt

European Patent Office

Office européen des brevets

(11) Veröffentlichungsnummer: **0 257 386 B1**

# EUROPÄISCHE PATENTSCHRIFT

(45) Veröffentlichungstag der Patentschrift: 30.10.91

(51) Int. Cl.⁵: **A61K 9/48**

(21) Anmeldenummer: 87111310.6

(22) Anmeldetag: 05.08.87

(54) **Gelatinekapseln und Verfahren zu ihrer Herstellung.**

(30) Priorität: 29.08.86 DE 3629386

(43) Veröffentlichungstag der Anmeldung:
02.03.88 Patentblatt 88/09

(45) Bekanntmachung des Hinweises auf die
Patenterteilung:
30.10.91 Patentblatt 91/44

(84) Benannte Vertragsstaaten:
**AT BE CH DE ES FR GB GR IT LI LU NL SE**

(56) Entgegenhaltungen:
**BE-A- 861 612**
**DE-A- 3 500 103**
**GB-A- 2 155 789**

(73) Patentinhaber: **R.P. Scherer GmbH**
**Gammelsbacher Strasse 2 Postfach 1243**
**W-6930 Eberbach/Baden(DE)**

(72) Erfinder: **Brox, Werner**
**Kätchen-Paulus-Strasse 6**
**W-6124 Beerfelden(DE)**

(74) Vertreter: **Werner, Hans-Karsten, Dr. et al**
**Deichmannhaus am Hauptbahnhof**
**W-5000 Köln 1(DE)**

EP 0 257 386 B1

**Beschreibung**

Gegenstand der vorliegenden Erfindung sind Gelatinekapseln bestehend aus einer Hülle aus Gelatine und gegebenenfalls einem Weichmacher und aus einer Kapselfüllung, die mindestens einen Wirkstoff und/oder ein Diät- oder Lebensmittel sowie eine Lösungsmittelmischung enthält, sowie Verfahren zur Herstellung derselben.

Weichgelatinekapseln dienen vorwiegend zur Aufnahme von Flüssigkeiten, wobei die Wirkstoffe in gelöster oder suspendierter Form vorliegen. Als Füllmaterialien sind pflanzliche, tierische und mineralische Öle, flüssige Kohlenwasserstoffe, etherische Öle und Polyethylenglykol im Gebrauch. Zur Konsistenzerhöhung werden auch Fette und Wachse verwendet oder zugesetzt.

In den letzten Jahren sind auch Verfahren entwickelt worden, Flüssigkeiten und pastöse Füllmaterialien in Hartgelatinekapseln abzufüllen.

Eine besonders gute Bioverfügbarkeit der pharmakologisch aktiven Wirkstoffe wird erreicht, wenn es gelingt, den Wirkstoff in einem geeigneten Lösungsmittel zu lösen und die verkapselte Lösung dem Patienten zu verabreichen. Als Lösungsmittel kommen jedoch nur solche Hilfsstoffe in Betracht, die einerseits für Humanzwecke unbedenklich sind und andererseits die Stabilität der Gelatinehülle nicht beeinträchtigen. Aus der DE-OS 33 07 353 der Anmelderin sind Weichgelatinekapseln bekannt, die als Lösungsmittel Polyethylenglykole mit einem mittleren Molekulargewicht von 600 sowie Glycerin und/oder 1,2 Propylenglykol enthalten. Diese Weichgelatinekapseln haben sich sehr bewährt. Die Polyethylenglykole sind jedoch für verschiedene Wirkstoffe als Lösungsmittel ungeeignet. Es gibt empfindliche Wirkstoffe, beispielsweise Sulfonamide oder organische Jodverbindungen, die in Polyethylenglykolen chemisch instabil sind. Ferner sind eine Reihe von Wirkstoffen bekannt, beispielsweise Phenole oder Phenobarbital, bei denen zwischen den Ethoxylgruppen der Polyethylenglykole und den Wirkstoffen Komplexe gibildet werden, welche eine verringerte Bioverfügbarkeit der Wirkstoffe verursachen. Schließlich gibt es höher dosierte Wirkstoffe, beispielsweise Pentetrazol, die sich in Polyethylenglykolen nicht gut genug lösen. Aufgabe der Erfindung war es, nach Lösungsmitteln zu suchen, die für Humanzwecke erlaubt sind, ein gutes Lösevermögen haben, stabil verkapselt werden können und keine unerwünschten Komplexe durch Ethoxylgruppen verursachen.

Ethanol ist als gutes Lösungsmittel für viele pharmazeutische Wirkstoffe bekannt und wird deshalb häufig zur Herstellung von Tropflösungen eingesetzt. Dagegen konnte Ethanol bisher als Lösungsmittel für einzel dosierte Gelatinekapseln in ausreichend hoher Konzentration nicht verwendet werden, weil konzentrierter Ethanol von der Gelatinehülle nach der Herstellung aufgenommen wird, die Gelatinehülle erweicht und deformiert, so daß solche Kapseln nicht handelsfähig sind. Nach den Angaben von Czetsch-Lindenwald und Fahrig, Arzneikapseln Aulendorf 1962, S. 34, können niedrige aliphatische Alkohole nur bis ca. 5% in Kapseln abgefüllt werden. Im Gemisch mit Polyethylenglykolen soll in einer speziellen Rezeptur gemäß DE-OS 35 09 741 maximal 10% Ethanol in Weichkapseln abgefüllt werden. Das Beispiel enthält jedoch nur 7% Ethanol. Dies wird bestätigt durch die Europäische Patentpublikation 0 170 623, welche für den Wirkstoff Dihydro-(val)$^2$-Cyclosporin Ethanol als Lösungsmittel verwendet. Trinklösungen enthalen 10 bis 12 % Ethanol, die Weichgelatinekapseln gemäß Beispiel 2 jedoch wiederum nur 2 bis 5 %.

Es wurde nun überraschenderweise gefunden, daß auch Ethanolmischungen mit einem Ethanolgehalt über 5 Gew.-% aber ohne Zusatz von Ethylenglykolen stabil verkapselt werden können, wenn das Lösungsmittelgemisch mindestens 20 Gew.-% Partialglyceride von Fettsäuren mit 6 bis 18 Kohlenstoffatomen enthält. Vorzugsweise werden Partialglyceride von Fettsäuren mit 6 bis 12 Kohlenstoffatomen und/oder der Ricinolsäure eingesetzt.

Als Partialglyceride können sowohl Mono- als auch Diglyceride sowie Gemische derselben eingesetzt werden. Geeignete Handelsprodukte sind beispielsweise das Glycerinmonocaprylat (Imwitor® 308 der Dynamit Nobel), das Glycerinmonodicaprylat (Imwitor® 908), Mischungen von Glycerinmondicaprylat und Glycerinmonodicaptrat (Imwitor® 742) und Partialglyceride der Ricinolsäure (Softigen® 701 und Rilanit®).

Diese Partialglyceride sind mit Ehtanol gut mischbar. Sie gestatten sogar Ethanolmengen von mehr als 10 Gew.-% in dem Lösungsmittelgemisch einzusetzen, ohne daß hierdurch die Stabilität der Gelatinehülle negativ beeinflußt wird.

Die erfindungsgemäßen Kapseln werden hergestellt, indem man den Wirkstoff in dem Gemisch von Ethanol und Partialglyceriden löst. Da die Ethanol-Partialglycerid-Mischungen sowohl mit lipophilen als auch hydrophilen Komponenten mischbar sind, können auch bei Bedarf verschiedene Zusätze z.B. Triglyceride verschiedener Fettsäuren oder Polyethylenglykole verwendet werden, sofern es die Stabilität der Wirkstoffe und der Gelatinekapsel erlaubt.

Die erfindungsgemäßen Lösungen können sowohl in Hartgelatinekapseln als auch in Weichgelatinekapseln abgefüllt werden. Die Kapselhülle von Weichgelatinekapseln enthält üblicherweise neben Gelatine

2

einen oder mehrere Weichmacher wie Glycerin, Propylenglykol, Sorbit, Sorbitane. Die Hülle kann auch Farbstoffe, Konservierungsstoffe, Geschmacksstoffe, Zucker und andere Polyole enthalten. Die folgenden Beispiele und Vergleichsbeispiele erläutern die Erfindung.

Biespiel 1

Die Mischung von 300 mg Glycerinmonocaprylat (Imwitor® 308) und 150 mg Ethanol wurde in Weichgelatinekapseln in bekannter Weise nach dem Rotary-Die-Verfahren gefüllt und die Kapseln getrocknet. Anschließend wurde der Ethanolgehalt der Kapselhülle analysiert und ein Ethanolgehalt von 4,5% gefunden. Die Kapseln waren physikalisch stabil; die Kapselform und Kapselhärte waren ausgezeichnet.

Vergleichsbeispiel 1

400 mg Ethanol wurden wie bei Beispiel 1 in Weichgelatinekapseln gefüllt. Nach dem Trocknen der Kapseln betrug der Ethanolgehalt der Kapselhülle 14,0%. Die Kapseln waren physikalisch instabil, deformiert und durch den Ethanolverlust geschrumpft.

Vergleichsbeispiel 2

Die Mischung von 350 mg mittelkettigen Triglyceriden (Miglyol® 812) und 100 mg Ethanol wurde wie bei Beispiel 1 in Weichgelatinekapseln gefüllt. Nach dem Trocknen der Kapseln betrug der Ethanolgehalt der Kapselhülle 11,8%. Die Kapseln waren physikalisch instabil. Sie waren weich und deformiert.

Beispiel 2

Die Mischung von 300 mg Glycerinmono/dioleat und 150 mg Ethanol wurde wie bei Beispiel 1 in Weichgelatinekapseln gefüllt. Nach dem Trocknen der Kapseln betrug der Ethanolgehalt der Kapselhülle 7,8%. Die Kapselform war zufriedenstellend, die Kapseln waren jedoch wegen des höheren Ethanolgehaltes in der Hülle weicher als bei Beispiel 1.

Beispiel 3

Hartgelatinekapseln der Größe O mit einem Durchschnittsgewicht von 97 mg wurden mit einer Mischung von 80% Glycerinmonocaprylat (Imwitor® 308) und 20% Ethanol befüllt und mit einem Gelatinefaden banderoliert. Nach 1 Woche wurde der Ehtanolgehalt der Kapselhülle analysiert. Die Kapselhüllen enthielten 2% Ethanol bezogen auf 97 mg Gelatinehülle.

Vergleichsbeispiel 3

Hartgelatinekapseln der Größe O wurden wie im Beispiel 3 mit einer Mischung von 80% mittelkettigen Triglyceriden (Miglyol® 812) und 20% Ethanol befüllt und mit einem Gelatinefaden banderoliert. Nach 1 Woche betrug der Ethanolgehalt der Kapselhülle 11,1% bezogen auf 97 mg Gelatinehülle.

Beispiel 4

| Pentetrazol | 100 mg |
| Ethanol | 20mg |
| Imwitor® 742 | 100 mg |
| Füllgewicht | 220 mg |

Zu dem geschmolzenen Inwitor 742 wurde Ethanol hinzugefügt und das Pentetrazol in der Mischung gelöst. Die fertige Lösung wurde in Weichgelatinekapseln der Größe 4 minims abgefüllt. Die getrockneten Kapseln waren einwandfrei bezüglich Kapselhärte und Kapselaussehen.

Beispiel 5

| Phenobarbital | 15,0 mg |
| Ethanol | 50,0 mg |
| Glycerinmonoricinoleat (Softigen® 701) | 150,0 mg |
| Füllgewicht | 215,0 mg |

Phenobarbital wurde in der Mischung von Ethanol und Glycerinmonoricinoleat gelöst und in Weichgelatinekapsein abgefüllt. Die Kapseln waren einwandfrei bezüglich Kapselhärte und Aussehen.

**Patentansprüche**

1. Gelatinekapseln bestehend aus einer Hülle aus Gelatine und gegebenenfalls einem Weichmacher und aus einer Kapselfüllung, die mindestens einen Wirkstoff und/oder ein Diät- oder Lebensmittel sowie eine Lösungsmittelmischung enthält, dadurch gekennzeichnet, daß das Lösungsmittelgemisch mindestens 5 Gew.-% Ethanol und mindestens 20 Gew.-% eines oder mehrerer Partialglyceride von Fettsäuren mit 6 bis 18 Kohlenstoffatomen enthält.

2. Gelatinekapseln nach Anspruch 1, dadurch gekennzeichnet, daß Partialglyceride von Fettsäuren mit 6 bis 12 Kohlenstoffatomen und/oder der Ricinolsäure eingesetzt werden.

3. Gelatinekapseln nach Anspruch 1 oder 2, dadurch gekennzeichnet, daß das Lösungsmittelgemisch mindestens 10 Gew.-% Ethanol enthält.

4. Verfahren zur Herstellung von Gelatinekapseln bestehend aus einer Hülle aus Gelatine und gegebenenfalls einem Weichmacher und aus einer Kapselfüllung, die mindestens einen Wirkstoff und/oder ein Diät- oder Lebensmittel sowie eine Lösungsmittelmischung enthält, dadurch gekennzeichnet, daß der Wirkstoff und/oder das Diät- oder Lebensmittel in einem Lösungsmittelgemisch, welches mindestens 5 Gew.-% Ethanol und mindestens 20 Gew.-% eines oder mehrerer Partialglyceride von Fettsäuren mit 6 bis 18 Kohlenstoffatomen enthält, gelöst wird und diese Lösung in an sich bekannter Weise in Gelatinekapseln abgefüllt wird.

**Claims**

1. Gelatin capsules, consisting of a shell comprising gelatin and optionally a plasticizer and a capsule filling containing at least one active substance and/or a dietetic agent or foodstuff as well as a solvent mixture, characterized in that the solvent mixture contains at least 5% by weight of ethanol and at least 20% of one or more of partial glycerides of fatty acids having from 6 to 18 carbon atoms.

2. Gelatin capsules according to claim 1, characterized in that partial glycerides of fatty acids having from 6 to 12 carbon atoms and/or of ricinoleic acid are used.

3. Gelatin capsules according to claims 1 or 2, characterized in that the solvent mixture contains at least 10% by weight of ethanol.

4. A process for preparing gelatin capsules, consisting of a shell comprising gelatin and optionally a plasticizer and a capsule filling containing at least one active substance and/or a dietetic agent or foodstuff as well as a solvent mixture, characterized in that the active substance and/or the dietetic agent or foodstuff are dissolved in a solvent mixture which contains at least 5% by weight of ethanol and at least 20% of one or more of partial glycerides of fatty acids having from 6 to 18 carbon atoms and the resulting solution is filled into gelatin capsules in a per se known manner.

**Revendications**

1. Capsule de gélatine constituée d'une enveloppe de gélatine avec éventuellement un agent amollissant, et d'un remplissage de capsule qui contient au moins une substance active ou une substance alimentaire ou diététique, ainsi qu'un mélange solvant,
caractérisée en ce que le mélange solvant contient au moins 5 % en poids d'éthanol et au moins 20 % en poids d'un ou plusieurs glycérides partiels d'acides gras comportant 6 à 18 atomes de carbone.

2. Capsule de gélatine selon la revendication 1, caractérisée en ce qu'elle contient des glycérides partiels d'acides gras comportant 6 à 12 atomes de carbone et/ou d'acide ricinoléique.

3. Capsule de gélatine selon la revendication 1 ou 2, caractérisée en ce que le mélange solvant comprend au moins 10 % en poids d'éthanol.

4. Procédé d'obtention de capsule de gélatine constituée d'une enveloppe de gélatine avec éventuellement un agent amollissant, et d'un remplissage de capsule qui contient au moins une substance active ou une substance alimentaire ou diététique, ainsi qu'un mélange solvant, caractérisé en ce qu'on dissout la substance active ou la substance alimentaire ou diététique dans un mélange solvant qui comprend au moins 5 % en poids d'éthanol et au moins 20 % en poids d'un ou plusieurs glycérides partiels d'acides gras comportant 6 à 18 atomes de carbone, et on remplit des capsules de gélatine, de manière connue, avec cette solution.